(19) Europäisches Patentamt
European Patent Office
Office européen des brevets

(11) EP 2 532 709 A1

(12) **EUROPEAN PATENT APPLICATION**
published in accordance with Art. 153(4) EPC

(43) Date of publication:
**12.12.2012 Bulletin 2012/50**

(21) Application number: **11739718.2**

(22) Date of filing: **31.01.2011**

(51) Int Cl.:
***C08L 27/12*** *(2006.01)* ***C07C 323/03*** *(2006.01)*
***C08F 214/18*** *(2006.01)* ***C08F 216/12*** *(2006.01)*
***C08K 5/37*** *(2006.01)* ***C08L 29/10*** *(2006.01)*
***G02B 1/04*** *(2006.01)*

(86) International application number:
**PCT/JP2011/051955**

(87) International publication number:
**WO 2011/096370 (11.08.2011 Gazette 2011/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.02.2010 JP 2010024702**

(71) Applicant: **Asahi Glass Company, Limited**
**Tokyo 100-8405 (JP)**

(72) Inventors:
- **SUGIYAMA, Norihide**
  **Tokyo 100-8405 (JP)**
- **MATSUURA, Keigo**
  **Tokyo 100-8405 (JP)**

(74) Representative: **Müller-Boré & Partner Patentanwälte**
**Grafinger Straße 2**
**81671 München (DE)**

(54) **CURABLE FLUORINATED RESIN COMPOSITION**

(57) To provide a fluorinated curable resin composition which is excellent in thermal stability and thermal adhesive property.

A fluorinated curable resin composition comprising a fluoropolymer (P) having a polymerizable double bond and a thiol compound (S), wherein the fluoropolymer (P) is a copolymer having repeating units derived from a fluoromonoene (a) and a fluorodiene (b) having a residual unsaturated side chain, and the content of the thiol compound (S) is from 0.01 to 1 part by mass per 100 parts by mass of the fluoropolymer (P).

Fig. 1

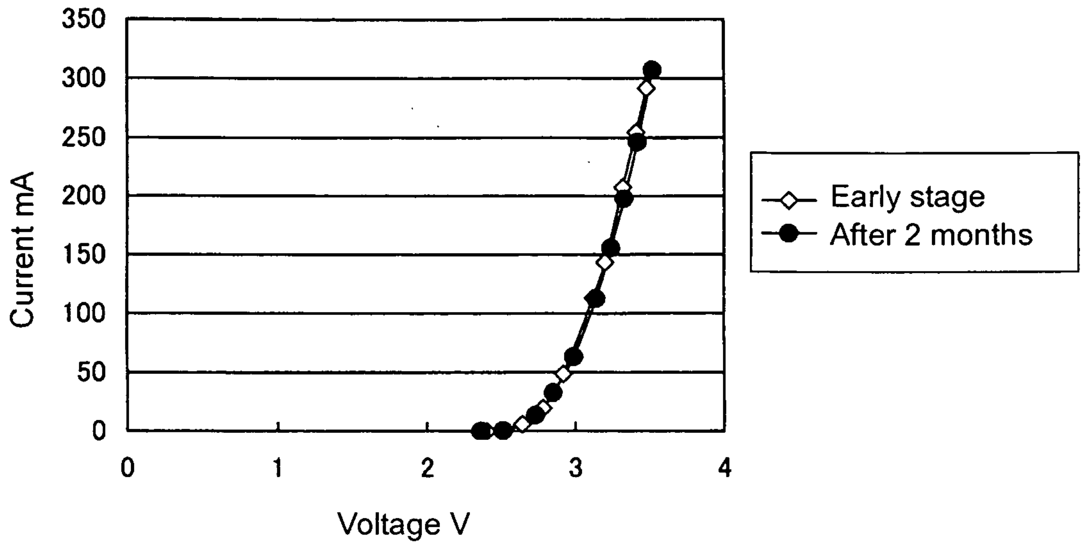


EP 2 532 709 A1

## Description

### TECHNICAL FIELD

The present invention relates to a fluorinated curable resin composition, a cured product thereof, an optical material and a light-emitting device using it and a novel fluorinated dithiol compound, specifically to a fluorinated curable resin composition whereby a cured product excellent in thermal stability and adhesive property can be obtained, a cured product thereof, an optical material and a light-emitting device using it and a novel fluorinated dithiol compound useful for the fluorinated curable resin composition.

### BACKGROUND ART

As a next generation high efficiency light-emitting device, a white LED (light emitting diode) lump is introduced, and has been developed to further improve luminance. White LED modules are encapsulated with a light-transmitting encapsulating resin such as an epoxy resin or a silicone resin to protect light-emitting devices. However, if the heating value in a light-emitting device increases as the electric power to be charged increases in future, deterioration of the light-transmitting encapsulating resin becomes problematic since the light-emitting device is subjected to high temperature. The deterioration of the light-transmitting encapsulating resin will decrease the light emitting output from the light-emitting device, and shorten the lifetime as a light source.
In order to solve the above problem, a method has been proposed in which a curable transparent fluororesin is employed (Patent document 1). Such a curable transparent fluororesin has a fluidity without containing a volatile component, and its cured product is excellent in thermal resistance and light resistance and free from coloration due to deterioration, and thereby preferably used to encapsulate power LED in a light-transmitting manner.

### PRIOR ART DOCUMENTS

### PATENT DOCUMENTS

Patent Document 1: W02009/096342

### DISCLOSURE OF INVENTION

### TECHNICAL PROBLEM

However, a cured product of a curable transparent fluorinated resin is not always excellent in long term stability. It is required to further improve properties in reliability tests such as a thermal cycle test, and it is necessary to solve problems such that in the thermal cycle test, a light-transmitting encapsulating resin peels, and thereby lighting defection results. Further, recently, LED lamps for illumination are required to have a high output, and gold is used as a wiring material to prevent the output from dropping due to deterioration. In such a case, a poor adhesive property of an encapsulating resin becomes problematic.
Accordingly, it is an object of the present invention to provide a fluorinated curable resin composition, whereby a cured product which is excellent in thermal stability and adhesive property and suitable for a light-transmitting encapsulating material for high power LED, can be obtained. Further, it is an object of the present invention to provide a cured product of the above fluorinated curable resin composition which is excellent in thermal stability and adhesive property, an optical material made of the cured product and a light-emitting device which is encapsulated in a light-transmitting manner with the cured product and has a high reliability in that it can be used for a long time.

### SOLUTION TO PROBLEM

[1] A fluorinated curable resin composition comprising a fluoropolymer (P) having polymerizable double bonds and a thiol compound (S), wherein the fluoropolymer (P) is a copolymer having repeating units derived from a fluoromonoene (a) and a fluorodiene (b) having a residual unsaturated side chain, and the content of the thiol compound (S) is from 0.01 to 1 part by mass per 100 parts by mass of the fluoropolymer (P).
[2] The curable composition according to the above [1], wherein the fluoromonoene (a) is at least one fluoromonoene selected from the group consisting of tetrafluoroethylene, chlorotrifluoroethylene and $CF_2$=CFO-Rf (wherein Rf is a $C_{1-6}$ fluoroalkyl group, and the fluoroalkyl group may have an etheric oxygen atom between carbon atoms).

[3] The curable composition according to the above [2], wherein Rf is a $C_{1-6}$ perfluoroalkyl group which may have an etheric oxygen atom between carbon atoms.
[4] The curable composition according to any one of the above [1] to [3], wherein the fluorodiene (b) is a fluorodiene represented by $CF_2$=CFO-Q-OCF=$CF_2$ (wherein Q is a $C_{3-8}$ fluoroalkylene group, and the fluoroalkylene group may have an etheric oxygen atom between carbon atoms).
[5] The curable composition according to the above [4], wherein Q is a $C_{3-8}$ perfluoroalkylene group which may have an etheric oxygen atom between carbon atoms.
[6] The curable composition according to the above [1], wherein the fluoropolymer (P) is a copolymer of tetrafluoroethylene, at least one perfluoroether represented by $CF_2$=CFO-$Rf^1$ (wherein $Rf^1$ is a $C_{1-6}$ perfluoroalkyl group, and the perfluoroalkyl group may have an etheric oxygen atom between carbon atoms) and at least one perfluorodiene represented by $CF_2$=CFO-$Q^1$-OCF=$CF_2$ (wherein $Q^1$ is a $C_{3-8}$ perfluoroalkylene group, and the perfluoroalkylene group may have an etheric oxygen atom between carbon atoms).
[7] The curable composition according to any one of the above [1] to [6], wherein the mass average molecular weight (Mw) of the fluoropolymer (P) is from 3,000 to 20,000.
[8] The curable composition according to any one of the above [1] to [7], wherein the content of polymerizable double bonds in the fluoropolymers (P) is from 0.1 to 2 mmol/g.
[9] The curable composition according to any one of the above [1] to [8], wherein the thiol compound (S) is a thiol compound having a melting point of from 50 to 100°C.
[10] The curable composition according to any one of the above [1] to [9], wherein the thiol compound (S) is a fluorinated thiol compound.
[11] The curable composition according to the above [10], wherein the fluorinated thiol compound is a fluorinated dithiol compound represented by the following formula:

$$HS(CH_2)_{m1}(CF_2)_n(CH_2)_{m2}SH$$

wherein m1 and m2 are 1 or 2, n is from 2 to 10, and m1 and m2 may be the same or different.
[12] A cured product which is obtained by curing the fluorinated curable composition as defined in any one of the above [1] to [11].
[13] An optical material made of the cured product as defined in the above [12].
[14] A light-emitting device which is encapsulated in a light-transmitting manner with the cured product as defined in the above [12].
[15] A fluorinated dithiol compound represented by the following formula:

$$HS(CH_2)_{m1}(CF_2)_n(CH_2)_{m2}SH$$

wherein m1 and m2 are 1 or 2, n is from 2 to 10, and m1 and m2 may be the same or different.

## ADVANTAGEOUS EFFECTS OF INVENTION

A cured product having improved thermal stability and adhesive property can be obtained from the fluorinated curable resin composition of the present invention without impairing transparency and light resistance.
The cured product of the present invention has excellent thermal stability and adhesive property without impairing transparency and light resistance.
The optical material and the light-emitting device of the present invention have high reliability.

## BRIEF DESCRIPTION OF DRAWINGS

[Fig. 1] A graph illustrating the relationship of voltage and current at the time of applying electricity to an LED device in Examples.

## DESCRIPTION OF EMBODIMENTS

Now, embodiments of the fluorinated curable resin composition of the present invention will be described in detail.
In the present specification, the number average molecular weight is represented by Mn, the mass average molecular weight is represented by Mw, and the molecular weight distribution is represented by Mw/Mn.
Further, the mass average molecular weight (Mw) and the number average molecular weight (Mn) in the present specification are ones obtained as molecular weights calculated as PMMA (polymethyl methacrylate) by gel permeation

chromatography (GPC) using, as a solvent, $CF_2ClCF_2CHClF$ (manufactured by Asahi Glass Company, Limited, tradename; AK225cb, hereinafter referred to as AK225cb).

Further, unless otherwise specified, the cured product of the fluorinated curable resin composition of the present invention is simply referred to as the cured product.

[Fluorinated curable resin composition]

The fluorinated curable resin composition of the present invention comprises a fluoropolymer (P) having polymerizable double bonds and a thiol compound (S). The fluoropolymer (P) is a copolymer having repeating units derived from a fluoromonoene (a) and a fluorodiene (b) having a residual unsaturated side chain, and the content of the thiol compound (S) is from 0.01 to 1 part by mass per 100 parts by mass of the fluoropolymer (P).

Further, as the case requires, a polymerizable compound (Y), a curing agent or a photoinitiator for curing, or other additives may be added to the fluorinated curable resin composition of the present invention.

[Fluoropolymer (P)]

The fluoropolymer (P) of the present invention has polymerizable double bonds (carbon-carbon double bonds). Since the fluoropolymer (P) has polymerizable double bonds in its molecule, it can be cured when the polymerizable double bonds are crosslinked by light or heat.

The fluoropolymer (P) of the present invention has repeating units derived from a fluoromonoene (a) and repeating units derived from a fluorodiene (b) having a residual unsaturated side chain (hereinafter referred to simply as fluorodiene (b)).

(Fluoromonoene (a))

Fluoromonoene (a) is a fluorinated compound having a polymerizable double bond in its molecule.

The fluoromonoene (a) may, for example, be a fluoroethylene such as tetrafluoroethylene, trifluoroethylene, chlorotrifluoroethylene or vinylidene fluoride; hexafluoropropylene; a fluorovinyl ether represented by $CF_2$=CFO-Rf (wherein, Rf is a $C_{1-6}$ fluoroalkyl group, and the fluoroalkyl group may have an etheric oxygen atom between carbon atoms); or a cyclic fluoromonomer represented by the following formula (a-1) or the following formula (a-2). Particularly, in a case where the fluorovinyl ether represented by $CF_2$=CFO-Rf (wherein, Rf is a $C_{1-6}$ fluoroalkyl group, and the fluoroalkyl group may have an etheric oxygen atom between carbon atoms) is used as the fluoromonoene (a), a fluoropolymer (P) to be obtained has a low viscosity, and a cured product to be obtained has high flexibility, and thus such a cured product is useful as a material for encapsulating a high power LED in a light-transmitting manner. Further, in a case where the cyclic fluoromonomer is used as the fluoromonoene (a), the glass transition temperature of a cured product to be obtained will be high, whereby the hardness becomes high, and such a cured product is useful as a lens, etc.

From the viewpoint of the thermal stability, the fluoromonoene (a) is preferably a perfluoromonomer, more preferably tetrafluoroethylene. Particularly, when tetrafluoroethylene is used as the fluoromonoene (a), the fluidity and the thermal stability of a fluoropolymer (P) to be obtained are the best. Further, more preferably, tetrafluoroethylene and a fluorovinyl ether represented by $CF_2$=CFO-Rf (wherein Rf is a $C_{1-6}$ fluoroalkyl group and the fluoroalkyl group may have an etheric oxygen atom between carbon atoms) are used in combination, whereby the fluidity is more improved. Further preferably, tetrafluoroethylene and a perfluorovinyl ether represented by $CF_2$=CFO-$Rf^1$ (wherein $Rf^1$ is a $C_{1-6}$ perfluoroalkyl group, and the perfluoroalkyl group may have an etheric oxygen atom between carbon atoms) are used in combination. Further, when chlorotrifluoroethylene is used as the fluoromonoene (a), the refractive index can be increased. By using a certain amount of chlorotrifluoroethylene, the refractive index can be increased by about 0.03 to 0.1. Accordingly, the output of LED light is improved.

When tetrafluoroethylene is used, the proportion of repeating units derived from tetrafluoroethylene is preferably from 1 to 80 mol%, particularly preferably from 50 to 70 mol%, per the total amount of repeating units derived from the fluoromonoene (a) and repeating units derived from the fluorodiene (b). When the proportion is within the above range, the thermal stability and the transparency of a cured product and the fluidity of the fluoropolymer (P) will be excellent.

When tetrafluoroethylene and $CF_2$=CFO-$Rf^1$ are used in combination, the proportion of repeating units derived from $CF_2$=CFO-$Rf^1$ is preferably from 1 to 49 mol%, particularly preferably from 10 to 40 mol%, per the total amount of repeating units derived from tetrafluoroethylene and $CF_2$=CFO-$Rf^1$.

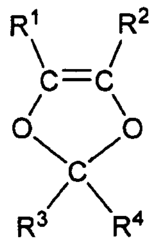

(a-1)

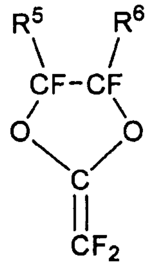

(a-2)

wherein, each of $R^1$ and $R^2$ which are independent of each other is a fluorine atom or an $OCF_3$ group, and each of $R^3$ and $R^4$ which are independent of each other is a fluorine atom or a $CF_3$ group. Further, each of $R^5$ and $R^6$ which are independent of each other is a fluorine atom, a perfluoroalkyl group, a perfluoroalkoxy group or a perfluoro(alkoxyalkyl) group.

(Fluorodiene (b))

The fluorodiene (b) is a fluorinated compound having 2 polymerizable double bonds in its molecule. The fluorodiene (b) is a compound having two polymerizable double bonds, in which at least a part of the double bonds does not contribute to the polymerization reaction and remains as the double bond after the polymerization. That is, two carbon atoms in one of the double bonds of the fluorodiene (b) form the main chain after the polymerization. At least a part of the other double bond does not contribute to the polymerization reaction and forms an unsaturated side chain having a polymerizable double bond in the fluoropolymer (P). By use of the fluorodiene (b), unsaturated side chains remain in the fluoropolymer (P), and accordingly a fluorinated cured product can be obtained by the curing reaction utilizing the unsaturated side chains.

The fluorodiene (b) may be a perfluorodiene consisting solely of carbon atoms and fluorine atoms or consisting solely of carbon atoms, fluorine atoms and oxygen atoms. Further, a fluorodiene having one or two fluorine atoms in the above perfluorodiene substituted by a hydrogen atom may be mentioned. The fluorodiene (b) is preferably a perfluorodiene in view of the thermal stability. In view of the fluidity and the thermal stability, a perfluorodiene consisting solely of carbon atoms, fluorine atoms and oxygen atoms is more preferred.

The fluorodiene (b) has preferably from 5 to 10, more preferably from 5 to 8 atoms in the connecting chain connecting the two polymerizable double bonds.

When the number of atoms in the connecting chain is at least the lower limit of the above range, intramolecular cyclization by reaction of the two polymerizable double bonds at the time of the polymerization reaction can be suppressed, whereby unsaturated side chains having a polymerizable double bond are likely to remain in the fluoropolymer (P). Further, when the number of atoms in the connecting chain is at most the upper limit of the above range , it is easy to prevent the fluoropolymer (P) from having a high molecular weight or from being gelated due to the crosslinking reaction by the polymerizable double bonds remaining in the side chains of each fluoropolymer (P) before the curing. Accordingly, it will be easy to prevent the fluidity before curing the curable composition from being remarkably decreased. Further, it is difficult to synthesize a fluorodiene (b) having a too long connecting chain and to purify it to have a high purity.

The fluorodiene (b) may be a fluoro cyclic diene having an alicyclic structure in its molecule or may be a fluoro acyclic diene having no alicyclic structure. Among them, the fluorodiene (b) is preferably a fluoro acyclic diene having no alicyclic structure, since it has a high effect to impart flexibility to a cured product to be obtained, and the fluidity will not be decreased too much.

<Fluoro acyclic diene>

The fluoro acyclic diene is a compound having no alicyclic structure as mentioned above. Further, the connecting chain connecting the two polymerizable double bonds is preferably a linear structure having no cyclic structure with a view to preventing the fluidity from being decreased too much.

The fluoro acyclic diene is preferably a compound represented by the following formula.

$$CF_2=CFO-Q^{F1}-OCF=CF_2$$

$$CF_2=CFOCH_2-Q^{F2}-CH_2OCF=CF_2$$

$CH_2=CFCF_2O-Q^{F3}-OCF_2CF=CH_2$

$CH_2=CFCF_2O-Q^{F4}-OCF=CF_2$

wherein each of $Q^{F1}$, $Q^{F2}$, $Q^{F3}$ and $Q^{F4}$ which are independent of one another, is a fluoroalkylene group which may have a side chain of a fluoroalkyl group, and the fluoroalkylene group may have an etheric oxygen atom between carbon atoms. The number of carbon atoms in the fluoroalkylene group represented by $Q^{F1}$ or $Q^{F3}$ is from 3 to 8, preferably from 3 to 6. The number of carbon atoms of the fluoroalkylene group represented by $Q^{F2}$ is from 2 to 6, preferably from 2 to 4. The number of carbon atoms in the fluoroalkylene group represented by $Q^{F4}$ is from 1 to 6, preferably from 2 to 5.

Among the above, from the viewpoint of an appropriate polymerization property for leaving a polymerizable double bond in a side chain at the time of preparing the fluoropolymer (P) and the viewpoint of the thermal stability of a cured product, preferred is a compound represented by $CF_2=CFO-Q-OCF=CF_2$ (wherein Q is a $C_{3-8}$ fluoroalkylene group, preferably, a $C_{3-6}$ fluoroalkylene group, and the fluoroalkylene group may have an etheric oxygen atom between carbon atoms). Particularly preferred is a compound represented by $CF_2=CFO-Q^1$ -$OCF=CF_2$ (wherein $Q^1$ is a $C_{3-8}$ perfluoroalkylene group, preferably a $C_{3-6}$ perfluoroalkylene group, and the perfluoroalkylene group may have an etheric oxygen atom between carbon atoms).

As specific examples of the fluoro acyclic diene, compounds represented by the following formulae may be mentioned.

$CF_2=CFO(CF_2)_4OCF=CF_2$

$CF_2=CFO(CF_2)_5OCF=CF_2$

$CF_2=CFO(CF_2)_6OCF=CF_2$

$CF_2=CFO(CF_2)_4OCF(CF_3)CF_2OCF=CF_2$

$CF_2=CFOCH_2(CF_2)_2CH_2OCF=CF_2$

$CF_2=CFOCH_2(CF_2)_4CH_2OCF=CF_2$

$CH_2=CFCF_2OCF(CF_3)CF_2OCF=CF_2$

$CH_2=CFCF_2OCF(CF_3)CF_2OCF(CF_3)CF_2OCF=CF_2$

Such fluorodienes (b) may be used alone, or two or more of them may be used in combination.

<Fluoro cyclic diene>

The fluoro cyclic diene is a compound having one or two alicyclic structures. The alicyclic structure in the fluoro cyclic diene consists solely of carbon atoms or carbon atoms and oxygen atoms. The number of atoms constituting the alicyclic structure is preferably from 4 to 8, more preferably 5 or 6. A particularly preferred alicyclic structure is a 5-membered ring or a 6-membered ring having one or two oxygen atoms.

In a case where the fluoro cyclic diene has two alicyclic structures, such alicyclic structures may be connected by a single bond or a bivalent or higher valent connecting group, or may be condensed (including a case where one carbon atom is shared). The connecting group may, for example, be an oxygen atom, a perfluoroalkylene group (preferably having at most 8 carbon atoms), or a perfluoroalkylene group (preferably having at most 8 carbon atoms) having an etheric oxygen atom at one or both terminals or between carbon atoms.

To the carbon atom constituting the alicyclic structure, a substituent other than a fluorine atom may be bonded. The substituent may, for example, be preferably a perfluoroalkyl group having at most 15 carbon atoms, a perfluoroalkyl group having at most 15 carbon atoms and having at least one etheric oxygen atom between carbon atoms, a perfluoroalkoxy group having at most 15 carbon atoms, or a perfluoroalkoxy group having at most 15 carbon atoms and having at least one etheric oxygen atom between carbon atoms.

One or both carbon atoms in at least one of the two polymerizable double bonds of the fluoro cyclic diene, are a carbon atom constituting the alicyclic structure. That is, in the fluoro cyclic diene, a polymerizable double bond is formed by adjacent carbon atoms constituting the alicyclic structure, or a polymerizable double bond is formed by one carbon atom constituting the alicyclic structure and a carbon atom bonded to the carbon atom. In a case where the fluoro cyclic diene has two alicyclic structures, each alicyclic structure has one of the two polymerizable double bonds.

The number of all carbon atoms in the fluoro cyclic diene is preferably from 8 to 24, more preferably from 10 to 18 in view of its boiling point and the heat resistance of a cured product to be obtained.

Further, the fluoro cyclic diene is preferably a compound having two alicyclic structures, wherein each of the two alicyclic rings has a polymerizable double bond, more preferably a compound having two perfluoro(2-methylene-1,3-dioxolane) structures. Further, it is more preferably a compound, as represented by the following formula (b-1), having two perfluoro(2-methylene-1,3-dioxolane) structures connected by a single bond or a bivalent connecting group with their 4-positions as connecting positions (hereinafter referred to as a compound (b-1)), or a compound, as represented by the following formula (b-2), having two perfluoro(2-methylene-1,3-dioxolane) structures connected by single bonds or bivalent connecting groups with their 4- and 5-positions as connecting positions, and it is particularly preferably the compound (b-1).

Further, as another fluoro cyclic diene, a compound represented by the following formula (b-3) may be mentioned.

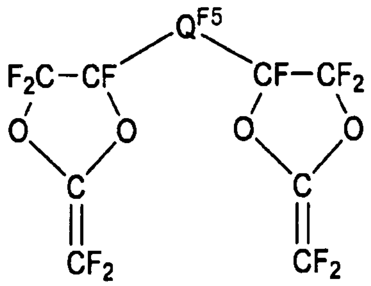

(b-1)

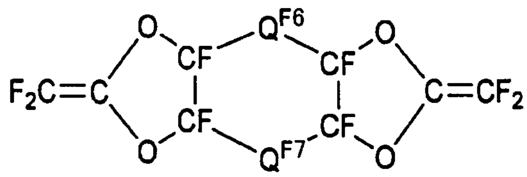

(b-2)

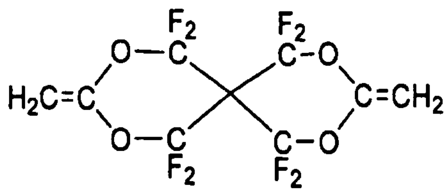

(b-3)

wherein $Q^{F5}$ is a single bond, an oxygen atom, or a perfluoroalkylene group having from 1 to 10 carbon atoms, which may have an etheric oxygen atom, and each of $Q^{F6}$ and $Q^{F7}$ which are independent of each other, is a single bond, an oxygen atom, or a perfluoroalkylene group having from 1 to 5 carbon atoms, which may have an etheric oxygen atom.

The polymerizable double bonds remaining in the side chains in the repeating units derived from the compound (b-1) are highly radical polymerizable. Accordingly, they are sufficiently reacted at the time of the curing reaction of the fluorinated curable resin composition, and remaining of side chains having a polymerizable double bond in a cured product to be obtained can be suppressed, whereby the thermal stability of the cured product will be improved.

As specific examples of the compound (b-1), compounds represented by the following formulae may be mentioned. The compound (b-1) is preferably prepared by a method disclosed in WO2005/085303.

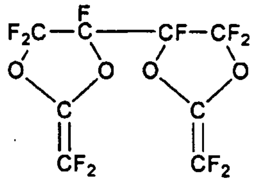

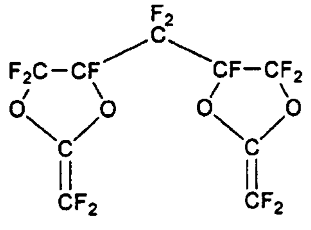

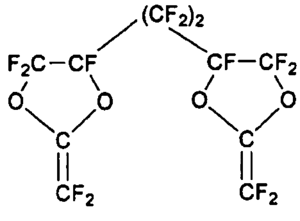

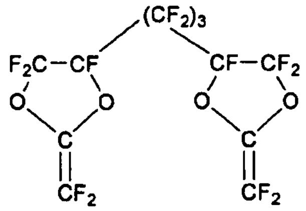
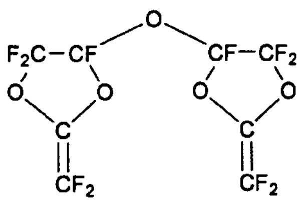
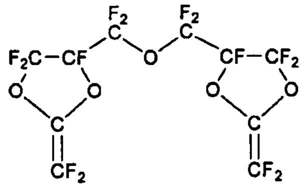

As an example of a preferred fluoropolymer (P), the following may be mentioned.

A copolymer of tetrafluoroethylene, at least one perfluoroether represented by $CF_2$=CFO-$Rf^1$ (wherein $Rf^1$ is a $C_{1-6}$ perfluoroalkyl group, and the perfluoroalkyl group may have an etheric oxygen atom between carbon atoms) and at least one perfluorodiene represented by $CF_2$=CFO-$Q^1$-OCF=$CF_2$ (wherein $Q^1$ is a $C_{3-8}$ perfluoroalkylene group, and the perfluoroalkylene group may have an etheric oxygen atom between carbon atoms).

The proportion of repeating units derived from the fluorodiene (b) is preferably from 1 to 95 mol%, more preferably from 1 to 30 mol%, particularly preferably from 5 to 15 mol%, per the total amount of repeating units derived from the fluoromonoene (a) and repeating units derived from the fluorodiene (b). When the proportion is higher than the above lower limit, crosslinking will be sufficient, whereby the thermal stability of a cured product will be excellent. When the proportion is lower than the above upper limit, side chains having a polymerizable double bond can be prevented from remaining, whereby the thermal stability of a cured product will be excellent.

(Preparation of fluoropolymer (P))

As described above, by copolymerizing the fluoromonoene (a) and the fluorodiene (b), a fluoropolymer (P) which is a copolymer in which unsaturated side chains having a polymerizable double bond remain in at least part of repeating units derived from the fluorodiene (b), is obtained.

For example, when $CF_2$=CF-O-$(CF_2)_4$-O-CF=$CF_2$ is used as the fluorodiene (b), the fluoropolymer (P) has at least repeating units represented by the following formula.

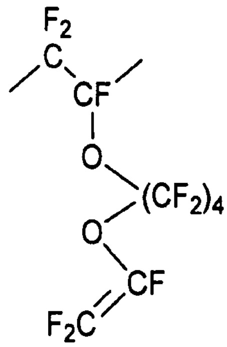

The fluoropolymer (P) can be obtained by copolymerizing the fluoromonoene (a) and the fluorodiene (b). The polymerization method of copolymerizing the fluoromonoene (a) and the fluorodiene (b) is not particularly limited, and a known polymerization method such as suspension polymerization, solution polymerization, emulsion polymerization or bulk polymerization may be employed. The solution polymerization is particularly preferred, since polymerization is possible in a state diluted with a solvent, and the crosslinking reaction between molecules by the polymerizable double bonds remaining in the side chains can be suppressed.

The solution polymerization is a polymerization method wherein the fluoromonoene (a) and the fluorodiene (b) are added to a polymerization initiator and copolymerized. Further, a chain transfer agent may be added.

The medium for polymerization in the solution polymerization is preferably a fluorinated solvent in which the fluoropolymers (P) to be formed are soluble. The fluorinated solvent may, for example, be dichloropentafluoropropane (HCFC-225), $CF_3CH_2CF_2H$ (HFC-245fa), $CF_3CF_2CH_2CF_2H$ (HFC-365mfc), perfluorohexane, perfluorooctane, perfluoro (2-butyltetrahydrofuran), perfluoro(tributylamine), $CF_3CF_2CF_2CF_2CF_2CF_2H$, $CF_3CH_2OCF_2CF_2H$, $CF_3CH_2OCH_2CF_3$ or $CF_3CF_2OCF_2CF_2OCF_2CF_3$.

Further, in production of the fluoropolymer (P), it is particularly preferred to conduct a step of preliminarily charging a part of the fluoromonoene (a) and the fluorodiene (b) to be used for preparation of the fluoropolymer (P) among the entire amount to be used, to a reactor to initiate the polymerization reaction, and successively adding the rest of the fluoromonoene (a) and the fluorodiene (b) during the progress of the polymerization reaction to conduct

polymerization, not to react the entire amount of the fluoromonoene (a) and the fluorodiene (b) all at once. By the above process, the molecular weight distribution and the composition distribution of the fluoropolymer (P) to be obtained can be made narrow, it will be easy to bring the content of low molecular weight components having a molecular weight of less than 1,000 among the fluoropolymer (P) to be less than 10 mass%, and the yield of the fluoropolymer (P) will be improved. Further, the fluoropolymer (P) include components having a particularly low fluorodiene (b) content and substantially not being a polymerizable compound in addition to low molecular weight components as polymerizable compounds, and it will be easy to reduce such compounds by the above step.

In production of the fluoropolymer (P), the molar ratio of the fluoromonoene (a) to the fluorodiene (b) is preferably from 40:60 to 95:5. Further, in a case where fluoroethylene is used as the fluoromonoene (a), the molar ratio of fluoroethylene to fluorodiene is more preferably from 50:50 to 95:5, particularly preferably from 70:30 to 95:5. If the proportion of the fluoromonoene (a) charged is too large, the molecular weight of the fluoropolymer (P) will be too high, and the fluidity will be decreased. Further, the transparency of a cured product to be obtained tends to be low.

<Polymerization initiator>

As a polymerization initiator to be used in the polymerization reaction, it is possible to use most of organic peroxides having a 10 hour half-life temperature being from 20 to 120°C, and it is preferred to use a fluorinated peroxide such as fluorinated diacyl peroxide so as to prevent a decrease in the reaction rate by a reaction to withdraw hydrogen atoms in the polymerization initiator.

The concentration of the polymerization initiator in the reaction solution is preferably from 0.1 to 5 mass%, more preferably from 0.5 to 2 mass%.

Further, the polymerization temperature varies depending on the 10 hour half-life temperature of the initiator and the polymerization rate of the monomers, and is preferably from 20 to 120°C, more preferably from 40 to 90°C.

<Chain transfer agent>

In the polymerization reaction, it is preferred to use a chain transfer agent.

The chain transfer agent may, for example, be a chlorine compound such as $CCl_4$, $CH_3Cl$, $SO_2Cl_2$ or $CHFCl_2$, or a hydrocarbon compound such as methanol, ethanol, isopropanol, hexane or diethyl ether. Particularly, $SO_2Cl_2$ is preferred since the chain transfer efficiency is high, and the fluoropolymer (P) can be obtained in high yield.

The amount of the chain transfer agent to be used varies depending on the chain transfer constant, but when $SO_2Cl_2$ is used, the amount is preferably from 0.001 to 0.1, more preferably from 0.001 to 0.05 by the molar ratio based on the total amount of the mixture of the fluoromonoene (a) and the fluorodiene (b). When the molar ratio is at least the above lower limit, it will be easy to prevent the molecular weight of the polymer from being too high. Further, when the molar ratio is at most the above upper limit, it will be easy to prevent the molecular weight of the fluoropolymer (P) from being decreased too much.

(Purification of fluoropolymer (P))

In a case where an obtained fluoropolymer (P) is used for a fluorinated curable resin composition, it is preferred to remove low molecular weight components having a molecular weight of less than 1,000.

It is known that polymerizable double bonds in the fluoropolymer (P) usually involve volume shrinkage when used for the polymerization reaction. It is considered that by removing compounds having a molecular weight of less than 1,000, the proportion of polymerizable double bonds per unit volume in the fluoropolymer (P) is made to be small, whereby the volume shrinkage of a cured product to be obtained can be suppressed, and the dimensional stability is improved.

A method of removing the low molecular weight components having a molecular weight of less than 1,000 may, for example, be a method of heating the fluoropolymer (P) under reduced pressure for removing, a method of extracting low molecular weight components from the fluoropolymers(P) by supercritical carbon dioxide, a method of charging a solution of the fluoropolymer (P) in a poor solvent to sediment the fluoropolymer (P), and removing the low molecular weight components which are not sedimented, or a method of fractionating and removing the low molecular weight components by means of gel permeation chromatography. A preferred method of removing the low molecular weight components is a method of heating the fluoropolymer (P) under reduced pressure for removing.

As conditions for removing components having a molecular weight of less than 1,000 by heating under reduced pressure, the pressure is preferably from 1 to 100 hPa, more preferably from 1 to 20 hPa, particularly preferably from 1 to 10 hPa. The temperature is preferably from 100 to 150°C, more preferably from 120 to 150°C. The lower the pressure (the higher the degree of vacuum), the better, but it is commonly not easy to reduce the pressure as the apparatus size increases. When the temperature is at least the above lower limit, a long period of time is not required to remove the

low molecular weight components. Further, if the temperature is at most the above upper limit, gelation reaction will not occur during heating.

A more preferred embodiment may be a method of reducing the content of low molecular weight substances contained in the fluoropolymer (P) by means of a method of heating under reduced pressure, and then removing low molecular weight substances by using an extraction solvent in a supercritical state to obtain the fluoropolymer (P).

After the fluoropolymer (P) is brought into contact with an extraction solvent in a supercritical state, the fluoropolymer (P) is separated from the extraction solvent, thereby to reduce the amount of the low molecular weight substances contained in the fluoropolymer (P).

The extraction solvent in the above extraction is a solvent in which the low molecular weight substances are dissolved, so that the low molecular weight substances and the fluoropolymer (P) can be separated.

The extraction solvent is not particularly limited so long as the above-described low molecular weight substances can be extracted at a temperature of the critical temperature of the extraction solvent to be used or higher and less than 130°C under a pressure of the critical pressure of the extraction solvent or higher. For example, carbon dioxide, or a fluorocarbon having from 1 to 3 carbon atoms such as fluoroform ($CF_3H$; R23) or perfluoroethane ($C_2F_6$; R116) may, for example, be mentioned. Among them, carbon dioxide, fluoroform or perfluoroethane is preferred, which is easily in a supercritical state, and is excellent in extraction efficiency, and carbon dioxide is more preferred.

The extraction solvents may be used alone or as a mixture of two or more, but by use of only one of carbon dioxide, fluoroform and perfluoroethane, the fluoropolymer (P) can be sufficiently purified.

The temperature of the extraction solvent in the extraction is a temperature of the critical temperature of the extraction solvent or higher and less than 130°C, and the pressure is the critical pressure of the extraction solvent or higher. That is, the above extraction is carried out by making the extraction solvent to be used be a supercritical fluid at a temperature less than 130°C and bringing it into contact with the fluoropolymer (P).

The above temperature may optionally be set in accordance with the extraction solvent to be used within the above range, and a preferred lower limit is a temperature higher than the critical temperature by 0.1°C, and a preferred upper limit is 100°C, more preferably 80°C.

The above pressure may optionally be set in accordance with the extraction solvent to be used within the above range, and a preferred lower limit is a pressure higher than the critical pressure by 10,000 Pa, and a preferred upper limit is a pressure higher than the critical pressure by 70 MPa.

In the above described purification method, by increasing the density of the extraction solvent such as carbon dioxide or fluoroform, the extraction efficiency of the low molecular weight substances can be improved. As the mechanism, an increase of the solubility of the low molecular weight substances in the extraction solvent by the increase of the density of the extraction solvent, is considered.

The density of the extraction solvent such as carbon dioxide or fluoroform is preferably at least 0.2 $g/cm^3$ and at most 1.3 $g/cm^3$ at the site of the extraction, i.e. under conditions where the extraction solvent is at the above described temperature under the above described pressure.

Further, as an auxiliary solvent, a halogenated hydrocarbon solvent or a hydrocarbon solvent (hereinafter referred to as an entrainer) may be used in combination with the extraction solvent in a supercritical state. The entrainers may be used alone or as a mixture. As specific examples of the fluorinated solvent to be used, the following compounds may be mentioned.

For example, $CF_3CF_2CHCl_2$, $CF_2ClCF_2CHClF$, $CF_3CF_2CHCl_2$, $CFCl_2CF_2Cl$, $CCl_4$, $CF_3CHFCHFCF_2CF_3$ or $CF_3CH_2OCF_2CF_2H$.

As the hydrocarbon solvent to be used, methanol, ethanol, propanol, isopropanol or dimethyl ether may, for example, be mentioned.

The above purification method is to carry out extraction by using an extraction solvent in a supercritical state, and accordingly the low molecular weight substances can efficiently be reduced, and the fluoropolymer (P) to be obtained has a narrow molecular weight distribution.

The above described purification method is to reduce the low molecular weight substances, and accordingly, the fluoropolymer (P) to be obtained has a narrower molecular weight distribution represented by Mw/Mn which is the ratio of the mass average molecular weight Mw to the number average molecular weight Mn measured by GPC.

(Fluoropolymer (P))

The fluoropolymer (P) preferably has a mass average molecular weight of from 3,000 to 20,000, more preferably from 5,000 to 15,000. The mass average molecular weights of the fluoropolymer (P) can be obtained as the molecular weight as calculated as PMMA (polymethyl methacrylate) by means of gel permeation chromatography (GPC).

When the mass average molecular weight of the fluoropolymer (P) is at least the above lower limit, volatilization of the low molecular weight components during the curing reaction of the curable composition tends to be prevented. Further, when the mass average molecular weight of the fluoropolymer (P) is at most the above upper limit, fluidity at

the minimum temperature at which the curing reaction takes place at the time of molding, or below, can be secured. If the molecular weight is too high and the fluidity is poor, molding into a desired shape cannot be carried out, or the fluidity tends to be non-uniform and accordingly there may be a deviation in characteristics of a molded product.

Further, when the mass average molecular weight of the fluoropolymer (P) is set high within the above range, a cured product having a higher thermal stability is likely to be obtained.

Further, the content of polymerizable double bonds remaining in the side chains in molecules of the fluoropolymer (P) is preferably from 0.1 to 2 mmol/g, more preferably from 0.2 to 0.5 mmol/g. The content of the polymerizable double bond can be calculated by measurement by $F^{19}$-NMR.

When the content of the polymerizable double bonds is at least the above lower limit, it will be easy to prevent a decrease in the hardness by insufficient crosslinking of a cured product to be obtained or to prevent a viscosity of a surface of the fluorinated curable resin composition from being too high. Further, when the content of the polymerizable double bonds is at most the above upper limit, it will be easy to prevent a too much decrease in the solubility in a solvent at the time of the polymerization reaction or in the solubility in a case of using a solvent at the time of the curing reaction, due to gelation by the crosslinking reaction between the fluoropolymer (P) molecules or by the polymer having a high molecular weight. Further, it will be easy to prevent a decrease in the thermal stability by remaining of unreacted polymerizable double bonds in the cured product to be obtained.

The fluoropolymer (P) has a high molecular weight and accordingly is a highly viscous liquid at room temperature, but when heated, its viscosity will be decreased, and it will have fluidity. The fluoropolymer (P) preferably has a viscosity of from 1 to 100 Pa·s at from 50 to 100°C.

Further, the fluoropolymer (P) will not substantially be cured at 100°C or below, and it is heat-cured at from 100 to 200°C, preferably from 150 to 200°C.

[Polymerizable compound (Y)]

The fluorinated curable resin composition of the present invention may contain another polymerizable compound (Y) in addition to the fluoropolymer (P). The polymerizable compound (Y) is a monomer having a molecular weight of at least 1,000 by itself or may be one which is polymerized to have a molecular weight of at least 1,000.

The polymerizable compound (Y) is preferably a fluoropolymer having a polymerizable double bond or a fluorooligomer having a polymerizable double bond, more preferably a perfluoropolymer having a polymerizable double bond or a perfluorooligomer having a polymerizable double bond. The polymerizable double bond in the perfluoropolymer or the perfluorooligomer is preferably one polymerizable double bond in the repeating units derived from the fluorodiene having a residual unsaturated side chain. A monomer constituting the perfluoropolymer having a residual unsaturated side chain or the perfluorooligomer having a residual unsaturated side chain may, for example, be $CF_2$=CFO-$Rf^2$-OCF=$CF_2$ or $CF_2$=CFO$CH_2$-$Rf^3$-$CH_2$OCF=$CF_2$.

In the formulae, each of $Rf^2$ and $Rf^3$ is a perfluoroalkylene group which may have a perfluoroalkyl group in its side chain or a group having an etheric oxygen atom between carbon atoms of the perfluoroalkylene group.

As specific examples of $Rf^2$ and $Rf^3$, for example, a perfluoropolyether having repeating units of e.g. -$CF_2$-, -$CF_2$O-, -$CF_2CF_2$O- -$CF_2CF_2CF_2$O- or -CF($CF_3$)$CF_2$O-may, for example, be mentioned.

A fluoropolymer having a polymerizable double bond or a fluorooligomer having a polymerizable double bond is obtained by polymerizing the above perfluorodiene and perfluoromonoene or the like. Here, the fluoropolymer having a polymerizable double bond and the fluorooligomer having a polymerizable double bond as the polymerizable compound (Y) are compounds other than the fluoropolymer (P) of the present invention.

[Thiol compound (S)]

The fluorinated curable resin composition of the present invention comprises a thiol compound (S). When the fluorinated curable resin composition comprises a thiol compound (S), the thermal stability and the adhesive property of a cured product can be improved. Further, the thiol compound means a compound having a mercapto group.

The reason why the thermal stability is improved by the thiol compound (S) is not clear, however, it is estimated as follows. A cured product has groups of which thermal stability is low such as -COOH or -$COOCH_3$ at a terminal of the main chain or a side chain of the fluoropolymer (P). If such a cured product is heated, the groups of which thermal stability is low decomposes, and then the main chain of the fluoropolymer (P) decomposes, and thereby the weight reduction of the cured produce results. This decomposition is a reaction in which radicals are formed. When the fluorinated curable resin composition comprises the thiol compound (S), H in mercapto groups is released, whereby the composition becomes stable, and the decomposition of the main chain of the fluoropolymer (P) is prevented. That is, it is considered that the thiol compound (S) has a function as a radical trapping agent. The function as the radical trapping agent can be represented as follows.

$$\sim\sim CF_2COOH \rightarrow \sim\sim CF_2\cdot\ \cdot COOH \sim\sim CF_2\ \cdot HS\sim\sim SH \rightarrow \sim\sim CF_2H\cdot S\sim\sim SH \sim\sim CF_2\cdot\ \cdot\ S\sim\sim SH \rightarrow \sim\sim CF_2S\sim\sim SH$$

Further, in the above reaction, a part of the thiol compound (S) bonds to the fluoropolymer (P), and a mercapto group is incorporated into a terminal or a side chain of the fluoropolymer (P), whereby an adhesive property to a substrate is improved by its polarity or a hydrogen bond property. Particularly, it is considered to be effective for gold used as a wiring material.

The thiol compound (S) is not particularly restricted, however, for easily mixing with the fluoropolymer (P), preferred is a compound having a melting point of from 50 to 100°C. When the thiol compound (S) has a melting point within the above range, the thiol compound (S) becomes liquid at a time of heat mixing with the fluoropolymer (P) and can be easily mixed.

The thiol compound (S) is preferably a fluorinated thiol compound, since it has a good solubility with the fluoropolymer (P).

The thiol compound (S) may, for example, be a thiol compound having an alkoxysilyl group or a thiol compound having no alkoxysilyl group.

(Thiol compound having an alkoxysilyl group)

As the thiol compound having an alkoxysilyl group, a compound containing the following formula:

$$(R_A)\ (R_B)\ (R_C)\ Si\text{-}$$

(wherein each of $R_A$, $R_B$ and $R_C$ which are independent of one another is a hydrogen atom or a $C_{1\text{-}5}$ alkoxy group) and having from 1 to 10 mercapto groups in its molecule, may be mentioned. In a case of having an alkoxysilyl group in its molecule, the adhesive property to a substrate is improved. Thus, in a case of having one mercapto group in its molecule, such a compound has a function as a radical trapping agent, and in a case of having at least 2 mercapto groups in its molecule, such a compound has a function as a radical trapping agent, and the adhesion property is improved. From the viewpoint of the adhesive property, a trialkoxysilyl group in which each of $R_A$, $R_B$ and $R_C$ is an alkoxy group is preferred. Further, the carbon number of the alkoxy group is from 1 to 5, and from the viewpoint of improving the reactivity, the carbon atom number is preferably from 1 to 3, particularly preferably from 1 to 2. The trialkoxysilyl group is preferably a trimethoxysilyl group or a triethoxysilyl group.

As another example of the thiol compound having an alkoxysilyl group, a compound represented by the following formula may be mentioned:

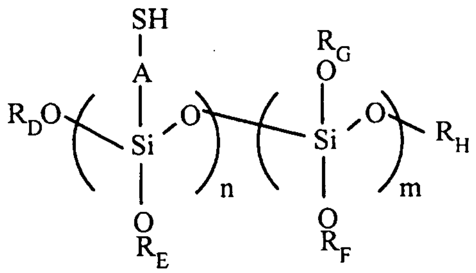

wherein each of $R_D$, $R_E$, $R_F$, $R_G$ and $R_H$ which are independent of one another is a $C_{1\text{-}5}$ alkyl group, A is a $C_{1\text{-}10}$ alkylene group or $C_{1\text{-}10}$ alkenylene group, and each of n and m which are independent of each other is an integer of from 1 to 10.

As specific examples of the thiol compound (S) having an alkoxysilyl group, KBM803 (tradename, manufactured by Shin-Etsu Chemical Co., Ltd.), X41-1810 (tradename, manufactured by Shin-Etsu Chemical Co., Ltd.) and X41-1805 (tradename, manufactured by Shin-Etsu Chemical Co., Ltd.) may be mentioned.

(Thiol compound having no alkoxysilyl group)

As the thiol compound having no alkoxysilyl group, a compound having from 2 to 10 mercapto groups in its molecule may be mentioned. Such a compound preferably has from 2 to 8 mercapto groups, more preferably from 2 to 5 mercapto groups. As specific examples of the thiol compounds having no alkoxysilyl group, an alkanedithiol such as 1,2-ethanedithiol, 2,3-butanedithiol, 1,6-hexanedithiol, 1,8-octanedithiol, 1,9-nonanedithiol, 1,10-decanedithiol, 1,11-undecanedithiol or 1,12-dodecanedithiol; a dithiol compound wherein an oxygen atom is introduced in an optional alkylene

group in the alkanedithiol such as an alkenedithiol having a polymerizable double bond at an optional position in an alkylene group in the above alkanedithiol, bis(2-mercaptoethyl)ether or 1,2-bis(2-mercaptoethoxy)ethane; a dithiol compound wherein an optional alkylene group in the above alkanedithiol is interrupted by a sulfur atom such as bis(2-mercaptoethyl)sulfide or 2,2'-(ethylene bisthio)bis(ethane thiol); a cycloalkanedithiol such as 1,3-cyclobutanedithiol, 1,4-cyclohexanedithiol or p-methane-2,9-dithiol; an aromatic dithiol such as benzene-1,4-dithiol, benzene-1,3-dithiol, 4,5-dimethyl-1,2-benzene bis(methanethiol), 1,4-benzene methanethiol, 2,6-naphthalene-dithiol, biphenyl-4,4'-bisthiol, 9H-purine-2,6-dithiol, tetrahydro-1H-1,2,4-triazole-3,5-dithione or 1,3,4-thiaziazole-2,5-dithiol; 1,1,1-butanetrithiol, ethene-1,1,2-trithiol, trithiocyanuric acid, 1,3,5-benzenetrithiol, triphenylene-1 ,5,9-trithiol, 2,2,5,5-hexanetetrathiol, tetrathiafulvalenetetrathiol, cyclobutadiene 1,2,3,4-tetrathiol, ethenetetrathiol, 1,3-butadiene-1,2,3,4-tetrathiol, 1,2,4,5-benzenetetrathiol, bibenzyl-2,2',3,3'-tetrathiol or 1,1',4',1"-terbenzene-3,3",4,4"-tetrathiol may be mentioned.

As commercially available products of the thiol compound having no alkoxysilyl group, EGMP-4 (tradename, manufactured by SC Organic Chemical Co., Ltd.), Karenz MTBD1 (registered trademark, manufactured by Showa Denko K.K.), TMMP (tradename, manufactured by SC Organic Chemical Co., Ltd.), TEMPIC (tradename, manufactured by SC Organic Chemical Co., Ltd.), Karenz MT NR1 (registered trademark, manufactured by Showa Denko K.K.), PEMP (tradename, manufactured by SC Organic Chemical Co., Ltd.) or Karenz MT PE1 (registered trademark, manufactured by Showa Denko K.K.) may be mentioned.

Particularly, preferred is a fluorinated dithiol compound having two functional groups represented by the following formula, since it is excellent in solubility with the fluoropolymer (P), and a cured product to be obtained is free from coloration:

$$HS(CH_2)_{m1}(CF_2)_n(CH_2)_{m2}SH$$

(wherein, m1 and m2 are 1 or 2. n is an integer of from 2 to 10. m1 and m2 may be the same or different from each other).

For example, the fluorinated dithiol compound represented by the above formula can be produced by substituting iodine atoms of the fluorinated diiodide compound represented by $I(CH_2)_{m1}(CF_2)_n(CH_2)_{m2}I$ by mercapto groups. The method for substituting by mercapto groups may, for example, be a method of reacting mercaptourea in the presence of a base.

The amount of the thiol compound (S) to be added is from 0.01 to 1 part by mass, preferably from 0.01 to 0.1 part by mass, per 100 parts by mass of the fluoropolymer (P). When the amount to be added is at least the lower limit of the above range, the desired effect of the present invention can be obtained, and when the amount to be added is at most the upper limit of the above range, a cured product to be obtained is less likely to be colored.

[Other additives]

To the fluorinated curable resin composition of the present invention, as a case requires, a curing agent for cure, a photoinitiator or other additives may be added in addition to the polymerizable compound (Y) and the thiol compound.

Such other additives may, for example, be a phosphor for an optical element, a dye or a light diffusing agent such as silica or alumina fine particles. Further, as additives for applications which require heat resistance and chemical resistance other than an optical material, various inorganic fillers, glass fibers, PTFE (polytetrafluoroethylene) particles may, for example, be mentioned.

In a case where zirconia nanoparticles, titania nanoparticles or the like are used as other additives, it is possible to increase the refractive index by from about 0.05 to about 0.15 depending on the amount to be added, while the transparency is maintained.

The fluorinated curable resin composition comprises from 0.01 to 1 part by mass of a thiol compound (c), per 100 parts by mass of the fluoropolymer (P), whereby a cured product to be obtained is excellent in thermal stability and adhesive property. Further, in a case where the fluorinated curable resin composition contains a fluoropolymer (P) of which a terminal of the main chain is amidated, a synergetic effect can be obtained in thermal stability and adhesive property of a cured product to be obtained. A terminal of the main chain of the fluoropolymer (P) is amidated by making the fluoropolymer (P) be in contact with an amidation agent such as ammonia or amine, and a method is preferred such that ammonia is directly blown in a solution containing the fluoropolymer (P).

[Cured product]

The cured product of the present invention is a cured product obtained by curing the fluorinated curable resin composition.

The cured product of the present invention has high light resistance (especially durability against short wavelength light having a wavelength of from 200 to 500 nm) and transparency and is excellent in thermal stability and

adhesive property.

(Production process of cured product)

The cured product of the present invention can be obtained by curing the fluorinated curable resin composition by heat or ultraviolet ray (UV).

By UV curing the fluorinated curable resin composition of the present invention, even in the presence of the thiol compound (S), the curing reaction proceeds without being impaired, whereby an effect is obtained such that even though a cured product to be obtained is exposed to a high temperature for a long time, the thermal decomposition reaction can be suppressed. On the other hand, if the fluorinated curable resin composition is cured by heat, curing is impaired due to the thiol compound (S), and a cured product which is excellent in thermal stability is less likely obtained. Accordingly, the fluorinated curable resin composition of the present invention is preferably cured by UV.

<Heat curing>

In a case where the fluorinated curable resin composition of the present invention is cured by heat, the curing temperature is preferably from 100 to 250°C, more preferably from 125 to 220°C, particularly preferably from 150 to 200°C.

When the curing temperature is at least the above lower limit, a cured product can be obtained in a short period of time, thus increasing the productivity. Further, when the curing temperature is at most the above upper limit, a cured product excellent in dimensional stability will easily be obtained.

The method of heat curing t is not particularly limited, and it may, for example, be a method of heating the fluorinated curable resin composition at from 50 to 100°C to make it flow, which is applied and then cured, or a method of applying it by using a solvent, followed by curing, and the former is preferred.

The curing reaction may be carried out in a multi-stage manner such that the temperature increases stepwise. In a case where the curing reaction is carried out in a multi-stage manner, the curing temperature may be set so that at least the maximum temperature is within the above range.

Further, in the heat curing reaction, a curing agent such as a fluorinated organic peroxide may be used. From the viewpoint of the thermal stability of a cured product, it is preferred not to use the curing agent. The fluorinated curable resin composition of the present invention can be cured, even when no curing agent is used, by heating. The fluorinated organic peroxide may, for example, be $(C_6F_5C(CO)O)_2$, $((CF_3)_3CO)_2$ or $C_6F_5C(CO)OOC(CH_3)_3$.

The mechanism of the crosslinking reaction in a case where no curing agent is used is not clearly understood, but oxygen dissolved in the fluoropolymer (P) becoming a radical source, a part of the structure in the fluoropolymer (P) being pyrolytically decomposed to generate radicals, or thermal coupling reaction of side chains $-CF=CF_2$ groups in the fluoropolymer (P), etc. are considered as factors.

<UV curing>

When the fluorinated curable resin composition of the present invention is cured by UV, the wavelength of UV (ultraviolet rays) is preferably from 150 to 400 nm, more preferably from 193 to 365 nm, most preferably from 248 to 365 nm.

When UV having a wavelength of from 150 to 300 nm is used, the fluorinated curable resin composition can be cured without using a photoinitiator, and when UV having a wavelength of from 300 to 400 nm is used, it is preferred to use a photoinitiator. The light source is not particularly restricted, however, in a case of UV having a wavelength of from 250 to 400 nm, a metal halide lamp or an electrodeless discharge lamp is used, and in a case of UV having a wavelength of 254 nm, 313 nm and 365 nm, a high-pressure mercury lamp or a low-pressure mercury lamp is used. Further, in a case of UV having a wavelength of 248 nm, a KrF eximer laser is used, in a case of UV having a wavelength of 193 nm, an ArF eximer laser is used, and in a case of UV having a wavelength of 157 nm, an $F_2$ laser is used.

The irradiation intensity, the irradiation time and the presence or the absence of a photoinitiator varies depending on the wavelength of UV. Curing may be carried out by irradiation at an irradiation intensity within a range of from 0.1 to 500 $mW/cm^2$ for about 1 minute to about 10 hours.

Here, the mechanism why curing proceeds without use of a photoinitiator when short wavelength ultraviolet rays of from 150 to 300 nm are used, is not clearly understood. According to structural analysis by $^{19}F$-NMR, absence of a cyclobutane ring to be caused by thermal coupling of $-CF=CF_2$ groups in the side chains of the fluoropolymer (P), in the cured product, was confirmed. Accordingly, it is suggested that polymerization of $-CF=CF_2$ groups in the fluoropolymer (P) proceeds. As an initiation source, it is considered that a terminal group having a carbonyl group such as -COOH present at the terminal of the fluoropolymer (P) undergoes removal of $CO_2$ by ultraviolet rays, or -COF formed by reaction of $O_2$ present in a very small amount with a $-CF=CF_2$ group undergoes removal of COF by ultraviolet rays, thereby to generate radicals (J. Fluorine Chemistry, (1987) Vol. 36, 449).

The photoinitiator may, for example, be an acetophenone compound, a benzoin ether compound, a benzyl

ketal compound, a ketone compound such as benzophenone or benzyl, an acylphosphine oxide compound, an O-acyloxime compound, a titanocene compound or a halomethyltriazine compound such as 2,4,6-tris(trichloromethyl)-1,3,5-triazine. Preferred is a fluorinated photoinitiator having some of hydrogen atoms substituted by fluorine or a fluoroalkyl group in view of compatibility with the fluoropolymer (P).

The amount of use of the photoinitiator is preferably from 0.01 to 10 parts by mass, more preferably from 0.1 to 1 part by mass, per 100 parts by mass of the total of the fluoropolymer (P) and the polymerizable compound (Y). When the amount of use of the photoinitiator is within the above range, a transparent cured product which is less colored is easily obtained without decreasing the curing rate.

[Optical material and light-emitting device]

The cured product obtained by curing the fluorinated curable resin composition of the present invention has high light resistance (especially durability against short wavelength light having a wavelength of from 200 to 500 nm) and transparency and is excellent in heat resistance, and is thereby useful as an optical material.

The optical material may be used for an application to a core material or clad material of optical fibers, a core material or clad material of an optical waveguide, a pellicle material, a surface protecting material for a display (e.g. PDP (plasma display panel), LCD (liquid crystal display), FED (field emission display) or an organic EL), a surface protecting material for a lens (e.g. a condensing lens for a light-emitting device, an artificial crystalline lens, a contact lens or a low refractive index lens), a material for a lens (e.g. a condensing lens for a light-emitting device, an artificial crystalline lens, a contact lens or a low refractive index lens), or a sealing material for producing a device (e.g. a light-emitting device, a solar cell device or a semiconductor device).

The optical material of the present invention is preferably used in optical applications as a molded product made of a cured product having an optional form (e.g. a plate-form, a tube form, a stick form, etc.) obtained by curing the fluorinated curable resin composition in an optional form of mold, or in optical applications as a coating film of the cured product to encapsulate an optional substrate in a light-transmitting manner, which is formed by curing the fluorinated curable resin composition or the like on an optional substrate (e.g. the above display, lens, device, etc.). Particularly, as a coating film used in optical applications, an application of a coating film which adhere to a substrate and through which light transmits is preferred. Further, the cured product of the present invention is preferably used in light-emitting devices for encapsulating a light-emitting chip in a substrate so that light emitted from the light-emitting chip can transmit through the sealing material and can be emitted from the devices, namely as a light transmitting sealing material.

The optical material used as the molded product is preferably a core material or clad material of optical fibers, a core material or clad material of an optical waveguide, or a material for a lens.

The coating film is preferably a sealing material for a device, for example, a sealing material to encapsulate a semiconductor device, a solar cell device or a light-emitting device (e.g. LED, laser diode (LD), an electroluminescence device, etc.) in a light-transmitting manner, and from the viewpoint that the fluorinated cured product of the present invention has the above properties, it is particularly preferably a sealing material to encapsulate a short wavelength light-emitting device in a light-transmitting manner. As the short wavelength light-emitting device, white LED may be mentioned.

As described above, the present invention provides a light-emitting device encapsulated in a light-transmitting manner with the optical material by using the fluorinated curable resin composition of the present invention. In a case where the light-emitting device of the present invention is a short wavelength light-emitting device having a wavelength of from 200 to 500 nm, it is possible to add e.g. a phosphor for changing a light-emitting wavelength of LED to the fluorinated curable resin composition, as the case requires. In case where a contained fluoropolymer (P) has a mass average molecular weight (Mw) of at most 20,000, the fluorinated curable resin composition of the present invention can easily flow when heated, whereby without using a solvent, a light-emitting device can be encapsulated by potting such a resin. Thus, the fluorinated curable resin composition of the present invention is excellent in thermal stability as an encapsulating resin. Further, since gold is used as a wiring material in some cases for high output light emitting devices such as LED lamps for illumination, the cured product of the present invention, which is excellent in adhesive property is useful as a encapsulating material.

EXAMPLES

Now, the present invention will be described in detail with reference to Examples and Comparative Example. However, it should be understood that the present invention is by no means restricted to the following Examples.

In Examples, the content of double bonds in the fluoropolymers (P) was measured by $^{19}F$-NMR. Further, the mass average molecular weight was obtained as a molecular weight as calculated as PMMA (polymethyl methacrylate) by means of gel permeation chromatography (GPC) using as a solvent $CF_2ClCF_2CHClF$ (manufactured by Asahi Glass Company, Limited, tradename: AK225cb, hereinafter referred to as AK225cb).

As a glass plate used in Examples, a transparent glass plate having a high transmission high transmittance to

UV of from 300 to 400 nm (made of a soda lime glass of which an iron content is low) was used.

Symbols of used materials are mentioned below.

<Monomer>

PPVE: Perfluoro(propyl vinyl ether) [$CF_2$=CFO-$CF_2CF_2CF_3$]
TFE: Tetrafluoroethylene [$CF_2$=$CF_2$]
C4DVE: Perfluoro(1,4-divinyloxybutane) [$CF_2$=CFO($CF_2$)$_4$OCF=$CF_2$]

<Thiol compound (S)>

TMMP (tradename, manufactured by SC Organic Chemical Co., Ltd.)
TEMPIC (tradename, manufactured by SC Organic Chemical Co., Ltd.)
KBM803 (tradename, Shin-Etsu Chemical Co., Ltd.)

[Synthesis Example 1: Preparation of fluoropolymer (P1)]

An autoclave made of stainless steel, having an internal capacity of 1 L, equipped with a stirrer, was deaerated, and then to this autoclave, PPVE (290 g) as a fluoromonoene (a), TFE (20 g), C4DVE (28 g) as a fluorodiene (b), AK225cb (600 g) and ($C_3F_7COO$)$_2$ (10 g) as a polymerization initiator were injected, and the interior of the autoclave was heated to 50°C with stirring. Then, TFE (total charged amount: 75 g) and C4DVE (total charged amount: 35 g) were successively added while the pressure was maintained under 0.2 MPa to carry out the polymerization reaction for 2 hours.

Then, the autoclave was cooled to room temperature, unreacted TFE was purged, and then the content was taken out and put in a glass beaker having an internal capacity of 2 L. To this glass beaker, 500 g of methanol was charged with stirring to precipitate a copolymer. The supernatant fluid was removed, and the precipitate was re-dissolved in AK225cb, and the solution was washed with water. Then, the lower layer was removed and subjected to filtration through a membrane filter made of PTFE having a pore size of 1 $\mu$m to obtain an almost transparent polymer solution. Then, the solvent of the obtained polymer solution was distilled off by means of an evaporator, and by heating in a vacuum at 120°C for 2 hours, 65 g of a colorless transparent and highly viscous liquid fluoropolymer (P1) was obtained.

The average molecular weight of the fluoropolymer (P1) was measured by GPC, whereupon the mass average molecular (Mw) weight was 11,500, and the number average molecular weight (Mn) was 6,100.

Further, the composition and the double bond content of the fluoropolymer (P1) were measured by $^{19}$F-NMR and as a result, the molar ratio of repeating units based on TFE, repeating units based on C4DVE and repeating units based on PPVE in the fluoropolymer (P1) was 67/6/27, and the double bond content was 0.22 mmol/g.

[Synthesis Example 2: Preparation example of fluorinated dithiol compound (S1)]

In a flask (capacity of 300 mL), $ICH_2CH_2CF_2CF_2CF_2CF_2CF_2CF_2CH_2CH_2I$ (4.9 g), thiourea (1.5 g) and ethanol (37 g) were added, and the mixture was stirred. The mixture was kept for about 11 hours under heating at 80°C.

Then, about 25 g of the ethanol in the flask was distilled off, about 20 g of a 20 wt% potassium hydroxide aqueous solution was added, and the mixture was heated to 90°C and stirred for 4 hours. Then, the mixture was stirred at room temperature overnight.

Then, about 23 g of a 2N hydrochloric acid was added, and being pH4, the mixture was stirred for 15 minutes. Then, about 54 g of AK-225 (a mixture of $CF_2ClCF_2CHClF$ and $CF_3ClCF_2CHCl_2$, manufactured by Asahi Glass Company, Limited) was added to extract the mixture. The aqueous phase was washed with AK-225, 6 times, AK-225 in the organic face was distilled off, and then the obtained liquid was purified by silica gel column chromatography to obtain 1.51 g of a compound (S1) the compound (S1) was analyzed by NMR and FT-IR. Results of analysis by NMR and FT-IR are mentioned below.
NMR data of the compound (S1).
$^1$H-NMR (282.7 MHz, solvent: $CDCl_3$, standard: TMS) $\delta$ (ppm): 1.6(2H), 2.4(4H), 2.8(4H).
$^{19}$F-NMR (282.7 MHz, solvent: $CDCl_3$ standard: $CFCl_3$) $\delta$ (ppm) : -115.1 (4F), -122.6(4F), -124.3(4F).

As a result of analysis by FT-IR, the absorption of the mercapto group was measured at around 2,550 $cm^{-1}$.

$$HS\text{-}CH_2CH_2\text{-}CF_2CF_2CF_2CF_2CF_2CF_2\text{-}CH_2CH_2\text{-}SH \qquad (S1)$$

[Example 1]

A frame produced by cutting out a silicon rubber sheet was put on a glass plate, and they were bonded. 100 parts by mass of the fluoropolymer (P1) prepared in Synthesis Example 1 and 0.1 part by mass of the fluorinated dithiol compound (S1) prepared in Synthesis Example 2 were mixed under heating at 100°C, the mixture was poured into the inside of the frame, followed by vacuum degassing, and then the frame was cooled. The frame was irradiated with UV by using a low-pressure mercury lamp UVB-40 (apparatus name, manufactured by SEN LIGHTS Co., Ltd., main wavelength: 254 nm) to cure the mixture, and thereby a colorless transparent rectangular cured product having a width of 1 cm, a length of 3 cm and a thickness of 1 mm was formed on the glass plate. With respect to the rectangular cured product, the following evaluations were carried out. Results are shown in Table 1.

<200°C heating test>

The rectangular cured product adhered on the glass plate was left in an oven at 200°C for 1,000 hours, and the weight reduction percentage (%) was measured. The smaller the weight reduction percentage (%) is, the better the thermal stability is.

<Adhesive property test>

An end of the rectangular cured product adhered on the glass plate was peeled from the glass plate at a rate of 15 mm/min, 90 degree peel strength from the glass plate was measured by a 90 degree peeling test machine (apparatus name, manufactured by Nisshin Kagaku Co., Ltd.).

[Comparative Example 1]

A cured product was prepared in the same manner as in Example 1, except that only the fluoropolymer (P1) prepared in Synthesis Example 1 was used, and the cured product was left in an oven at 200°C for 1,000 hours. The weight reduction percentage was 6.3%.

[Examples 2 to 4]

A rectangular cured product adhered on a glass plate was prepared in the same manner as in Example 1, except that instead of the fluorinated dithiol compound (S1) used in Example 1, a dithiol compound shown in Table 1 was used, and each evaluation was carried out. Results are shown in Table 1.

[Table 1]

| | | | Ex. 1 | Ex. 2 | Ex. 3 | Ex. 4 | Comp. Ex. 1 |
|---|---|---|---|---|---|---|---|
| Thiol compound(s) | | | Fluorinated dithiol compound (S1) | TMMP | TEMPIC | KBM803 | - |
| 200°C heating test | After heatingfor 10 hours | Colored | Not colored | Pale yellow | Pale yellow | Pale yellow | Not colored |
| | After heatingfor 50 hours | Colored | Not colored | Pale yellow | Pale yellow | Not colored | Not colored |
| | After heatingfor 100 hours | Colored | Not colored | Pale yellow | Not colored | Not colored | Not colored |
| | After heatingfor 200 hours | Colored | Not colored | Not colored | Not colored | Not colored | Not colored |
| | After heatingfor 1,000 hours | Weight reduction percentage (%) | 4.2 | 4.7 | 4.2 | 4.4 | 6.3 |
| | | Colored | Not colored | Not colored | Not colored | Not colored | Not colored |
| Adhesive property test (N/cm) | | | 1.0 | 0.6 | 0.5 | 0.8 | 0.4 |

[Example 5]

An LED device was encapsulated with the mixture prepared in Example 1, which comprises the fluoropolymer (P1) and the fluorinated dithiol compound (S1). Specifically, the mixture heated to 100°C was poured to a concave part of a cup type LED device (housing: made of alumina, electrode: gold) which is a wire bonded GaN LED (light emitting wavelength: 460 nm), followed by vacuum-degassing, and the LED device was cooled. The LED device was irradiated with UV by a low pressure mercury lamp UVB-40 (apparatus name: manufactured by SEN LIGHTS Co., Ltd.: main wavelength: 254 nm) to cure the mixture, and thereby the LED device was encapsulated.

Electric current was applied to the LED device, and the relationship of voltage-current shown in Fig. 1 was obtained. An electric current at 3.5 V and 350 mA was applied to the LED device, whereupon the electric current was not changed even after 2 months, and the transparency was maintained.

[Comparative Example 2]

An LED device was encapsulated in the same manner as in Example 5, except that only the fluoropolymer (P1) was used, and an electric current at 3.5 V was continuously applied to the LED device, whereupon the electric current was lowered after three weeks, and after 1 month, the LED device no longer emitted light.

It was measured that cured products prepared in Examples 1 to 4 were excellent in thermal stability and adhesive property. Particularly, the cured product of which the fluorinated curable resin composition contains the fluorinated dithiol compound (S1) prepared in Example 1 was colorless transparent after heating at 200°C for 1,000 hours. Such a cured product is useful as a LED encapsulating material which is required to prevent coloration. On the other hand, the cured product prepared in Examples 2 to 4 of which the fluorinated curable resin composition contains a thiol compound other than the fluorinated dithiol compound (S1) colored to pale yellow, depending on heating time.

Since the fluorinated curable resin composition containing a thiol compound was used for the LED device prepared in example 5, good voltage was maintained. On the other hand, since the fluorinated curable resin composition containing no thiol compound was used for the LED device prepared in Comparative Example 2, good voltage could

not be maintained.

INDUSTRIAL APPLICABILITY

According to the present invention, a fluorinated curable resin composition whereby a cured product which is suitable to an encapsulating material for high power LED in a light-transmitting manner, can be provided. Further, a cured product which is excellent in thermal stability and adhesive property, an optical material comprising the cured product and a light-emitting device encapsulated with the cured product and which is highly reliable can be provided.
The entire disclosure of Japanese Patent Application No. 2010-024702 filed on February 5, 2010 including specification, claims, drawings and summary is incorporated herein by reference in its entirety.

**Claims**

1. A fluorinated curable resin composition comprising a fluoropolymer (P) having polymerizable double bonds and a thiol compound (S), wherein the fluoropolymer (P) is a copolymer having repeating units derived from a fluoromonoene (a) and a fluorodiene (b) having a residual unsaturated side chain, and the content of the thiol compound (S) is from 0.01 to 1 part by mass per 100 parts by mass of the fluoropolymer (P).

2. The curable composition according to Claim 1, wherein the fluoromonoene (a) is at least one fluoromonoene selected from the group consisting of tetrafluoroethylene, chlorotrifluoroethylene and $CF_2$=CFO-Rf (wherein Rf is a $C_{1-6}$ fluoroalkyl group, and the fluoroalkyl group may have an etheric oxygen atom between carbon atoms).

3. The curable composition according to Claim 2, wherein Rf is a $C_{1-6}$ perfluoroalkyl group which may have an etheric oxygen atom between carbon atoms.

4. The curable composition according to any one of Claims 1 to 3, wherein the fluorodiene (b) is a fluorodiene represented by $CF_2$=CFO-Q-OCF=$CF_2$ (wherein Q is a $C_{3-8}$ fluoroalkylene group, and the fluoroalkylene group may have an etheric oxygen atom between carbon atoms).

5. The curable composition according to Claim 4, wherein Q is a $C_{3-8}$ perfluoroalkylene group which may have an etheric oxygen atom between carbon atoms.

6. The curable composition according to Claim 1, wherein the fluoropolymer (P) is a copolymer of tetrafluoroethylene, at least one perfluoroether represented by $CF_2$=CFO-$Rf^1$ (wherein $Rf^1$ is a $C_{1-6}$ perfluoroalkyl group, and the perfluoroalkyl group may have an etheric oxygen atom between carbon atoms) and at least one perfluorodiene represented by $CF_2$=CFO-$Q^1$-OCF=$CF_2$ (wherein $Q^1$ is a $C_{3-8}$ perfluoroalkylene group, and the perfluoroalkylene group may have an etheric oxygen atom between carbon atoms).

7. The curable composition according to any one of Claims 1 to 6, wherein the mass average molecular weight (Mw) of the fluoropolymer (P) is from 3,000 to 20,000.

8. The curable composition according to any one of Claims 1 to 7, wherein the content of polymerizable double bonds in the fluoropolymers (P) is from 0.1 to 2 mmol/g.

9. The curable composition according to any one of Claims 1 to 8, wherein the thiol compound (S) is a thiol compound having a melting point of from 50 to 100°C.

10. The curable composition according to any one of Claims 1 to 9, wherein the thiol compound (S) is a fluorinated thiol compound.

11. The curable composition according to Claim 10, wherein the fluorinated thiol compound is a fluorinated dithiol compound represented by the following formula:

$$HS(CH_2)_{m1}(CF_2)_n(CH_2)_{m2}SH$$

wherein m1 and m2 are 1 or 2, n is from 2 to 10, and m1 and m2 may be the same or different.

**12.** A cured product which is obtained by curing the fluorinated curable composition as defined in any one of Claims 1 to 11.

**13.** An optical material made of the cured product as defined in Claim 12.

**14.** A light-emitting device which is encapsulated in a light-transmitting manner with the cured product as defined in Claim 12.

**15.** A fluorinated dithiol compound represented by the following formula:

$$HS(CH_2)_{m1}(CF_2)_n(CH_2)_{m2}SH$$

wherein m1 and m2 are 1 or 2, n is from 2 to 10, and m1 and m2 may be the same or different.

Fig. 1

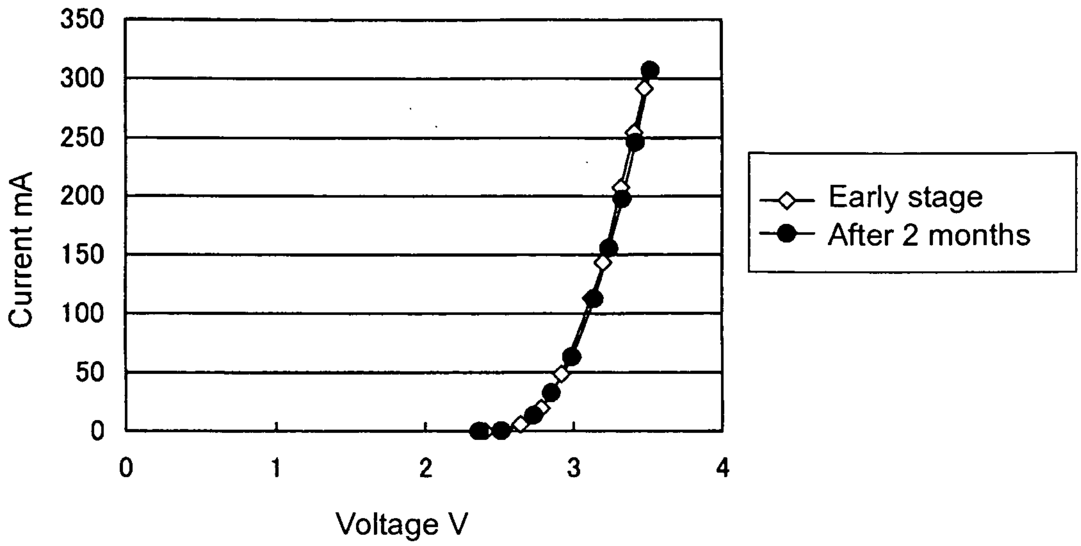

350
300
250
200
150
100
50
0
Current mA
0
1
2
3
4
Voltage V
Early stage
After 2 months

**INTERNATIONAL SEARCH REPORT**

International application No.
PCT/JP2011/051955

A. CLASSIFICATION OF SUBJECT MATTER
*C08L27/12*(2006.01)i, *C07C323/03*(2006.01)i, *C08F214/18*(2006.01)i, *C08F216/12*(2006.01)i, *C08K5/37*(2006.01)i, *C08L29/10*(2006.01)i, *G02B1/04*(2006.01)i

According to International Patent Classification (IPC) or to both national classification and IPC

B. FIELDS SEARCHED

Minimum documentation searched (classification system followed by classification symbols)
C08L27/12, C07C323/03, C08F214/18, C08F216/12, C08K5/37, C08L29/10, G02B1/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
WPI, CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>A | US 2961470 A (William A. sheppard),<br>22 November 1960 (22.11.1960),<br>example VI<br>(Family: none) | 15<br>1-14 |
| A | JP 2008-195929 A (Fujifilm Corp.),<br>28 August 2008 (28.08.2008),<br>entire text<br>& US 2009/0318650 A1 & WO 2008/087946 A1 | 1-15 |
| A | WO 2007/145181 A1 (Asahi Glass Co., Ltd.),<br>21 December 2007 (21.12.2007),<br>entire text<br>& US 2009/0118429 A1 & EP 2028200 A1 | 1-15 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>05 April, 2011 (05.04.11) | Date of mailing of the international search report<br>19 April, 2011 (19.04.11) |
| Name and mailing address of the ISA/<br>Japanese Patent Office<br>Facsimile No. | Authorized officer<br><br>Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009096342 A
- WO 2005085303 A
- JP 2010024702 A


### Non-patent literature cited in the description


- *J. Fluorine Chemistry,* 1987, vol. 36, 449